# EUROPÄISCHE PATENTSCHRIFT

(11) **EP 1 785 104 B1**
(45) Veröffentlichungstag und Bekanntmachung des Hinweises auf die Patenterteilung: **31.08.2011**
(21) Anmeldenummer: 05024779.0
(22) Anmeldetag: 14.11.2005
(51) Int. Cl.: A61B 19/02, A61G 12/00, A61G 13/00

(54) **Konsole mit zwei vertikalen Tragrohren**
Console with two uprights
Console avec deux montants verticaux

(43) Veröffentlichungstag der Anmeldung: 16.05.2007
(73) Patentinhaber: Ondal Holding GmbH, 36088 Hünfeld (DE)
(72) Erfinder: Knappe, Stefan, 36151 Burghaun (DE)
(74) Vertreter: Graf von Stosch, Andreas

(56) Entgegenhaltungen:
- WO-A-98/12093
- FR-A- 2 778 384
- US-A- 5 415 287

## Beschreibung

Der Anmeldegegenstand betrifft eine Konsole, insbesondere für den Klinikbereich. Eine solche Konsole ist aus FR 2 778 384 bekannt.

Es ist bekannt, im Klinikbereich Konsolen mit Tabletts zum Tragen von medizinischen Geräten zu nutzen. Dabei werden die Geräte auf den Tabletts angeordnet. Die elektrischen Anschlussleitungen der Geräte werden über die Tabletts gelegt und die Stecker der Anschlussleitungen werden in Steckdosen gesteckt. Die Tabletts sind einfach aufgebaut und werden entweder mittels eines Stecksystems auf eine bestimmte, vorgegebene Höhe gesteckt oder mittels einer relativ aufwändigen Montageeinrichtung auf einer bestimmten Höhe fixiert.

Es liegt die Aufgabe zugrunde, ein Tablett einer Konsole relativ leicht montierbar auf einer beliebigen Höhe an der Konsole zu befestigen.

Gelöst ist die Aufgabe gemäß Anspruch 1. Danach sind an der Konsole zwei vertikale Tragrohre vorgesehen. Es sind mindestens zwei Montageklauen vorgesehen, wobei die Montageklauen derart an den Tragrohren befestigt sind, dass zwei Montageklauen nebeneinander an den Tragrohren vorgesehen sind, und wobei an den Montageklauen jeweils eine Steckverbindung ausgebildet ist. Des weiteren ist mindestens einem Tablett vorgesehen, welches zwei Aufnahmen zum jeweiligen Aufnehmen einer Steckverbindung aufweist, um derart das Tablett auf die beiden Montageklauen stecken zu können.

Die vorgeschlagene Neuerung hat den Vorteil, dass das Tablett in einfacher Weise auf einer beliebigen Höhe fixiert werden kann. Die beiden Montageklauen, die das Tablett halten, können auf irgendeiner Höhe, die dem Zweck des auf dem Tablett befindlichen medizinischen Arrangements genügt, an den beiden Stangen montiert werden. Die Steckverbindungen werden in die Aufnahmen des Tabletts gesteckt. Somit ist die Montage des Tabletts durchgeführt.

Weitere, vorteilhafte Ausgestaltungen der vorgeschlagenen Neuerung sind in den Ansprüchen 2 bis 10 beschrieben.

Als Aufnahme, welche die Steckverbindungen und damit das Tablett stabilisiert, eignet sich ein Rechteckrohr (Anspruch 2).

Weist gemäß Anspruch 3 die Steckverbindung eine Kugel auf, die in der Steckverbindung gehalten wird, die mittels einer Feder gegenüber der Steckverbindung vorgespannt ist, und die zum Einrasten in eine Aussparung der Aufnahme vorgesehen ist, so werden zwei Vorteile erreicht. Zum einen hört man das Einrasten und erhält damit die Information, dass die Steckverbindungen in sicherer Weise in die Aufnahmen eingerastet sind. Zum anderen bietet die Kugel einen Widerstand, so dass eine Steckverbindung nicht zu leicht gelöst werden kann, es bei gezieltern Kraftaufwand jedoch zu einem einfachen Ablösen eines Tabletts von den Tragrohren kommt.

Besteht die Montageklaue aus zwei Klauenteilen, die jeweils ein Greifelement aufweisen, und wobei die Klauenteile mittels eines Verbindungselementes, vorzugsweise einer Schraube, miteinander verbunden sind (Anspruch 4), so können die Klauenteile zunächst getrennt mit dem Greifelement an ein Tragrohr gesetzt werden, wobei die Greifelemente das Tragrohr umgreifen. Nach Einsetzen und Befestigen des Verbindungselementes wird sodann eine sehr stabil am Tragrohr befestigte Steckverbindung erreicht.

Ist gemäß Anspruch 5 ein Sicherungselement, vorzugsweise eine Schraube, zum Sichern der Steckverbindung in der Aufnahme vorgesehen, so kann derart die Steckverbindung in ihrer Position in der Aufnahme gesichert werden.

Ein Gegenstand kann innerhalb eines Tabletts aufbewahrt werden, wenn das Tablett eine obere Wandung und eine untere Wandung aufweist, und die Aufnahmen zwischen den beiden Wandungen vorgesehen sind (Anspruch 6), Dann können die Räume zwischen den Wandungen als Stauraum genutzt werden (Anspruch 7). Ein derartiger Stauraum kann zur Aufnahme eines Elektrokabels, vorzugsweise mitsamt eines Netzsteckers vorgesehen werden (Anspruch 8). Damit wird erreicht, dass Kabel bzgl. ihrer überschüssigen Kabellänge sicher verwahrt werden. Stolperfallen durch Kabel oder Kabelschleifen werden somit vermieden.

Ist gemäß Anspruch 9 in der oberen Wandung, angrenzend an einen Stauraum, mindestens eine Aussparung zum Durchführen eines mit einer Anschlussbuchse versehenen Elektrokabels vorgesehen, so kann ein durch einen Stauraum geführtes Kabel (außer einem Elektrokabel auch eine Wasserversorgung oder eine Gasversorgung) ein auf dem Tablett befindliches Gerät versorgen. Am Tablett ist dabei nur das Kabelstück, welches von der Aussparung bis zum Gerät reicht, zu sehen. Mehrere Aussparungen dienen zum Durchführen mehrerer Kabel, Datenleitungen oder ähnlichem.

Ist an der Konsole ein Korpus vorgesehen, der eine Mehrzahl an Steckdosen aufweist, die über eine elektrische Versorgungsleitung mit elektrischem Strom versorgt werden (Anspruch 10), so kann für die Elektrokabel, die sich in den Stauräumen befinden, in kurzer Entfernung zu einem Tablett eine Elektrovessorgung erfolgen, ohne dass zu lange Kabelstrecken stören. Die elektrische Versorgungsleitung wird vorzugsweise von oben her zur Konsole geführt. Dies ist besonders vorteilhaft, da in einer bevorzugten Ausführungsform die Tragrohre an einem Deckenstativ hängen. Die Konsole könnte auch in einem Friseursalon eingesetzt werden.

Im folgenden wird die Erfindung an Hand ein Ausführungsbeispiel darstellender Figuren näher beschrieben. Es zeigt:

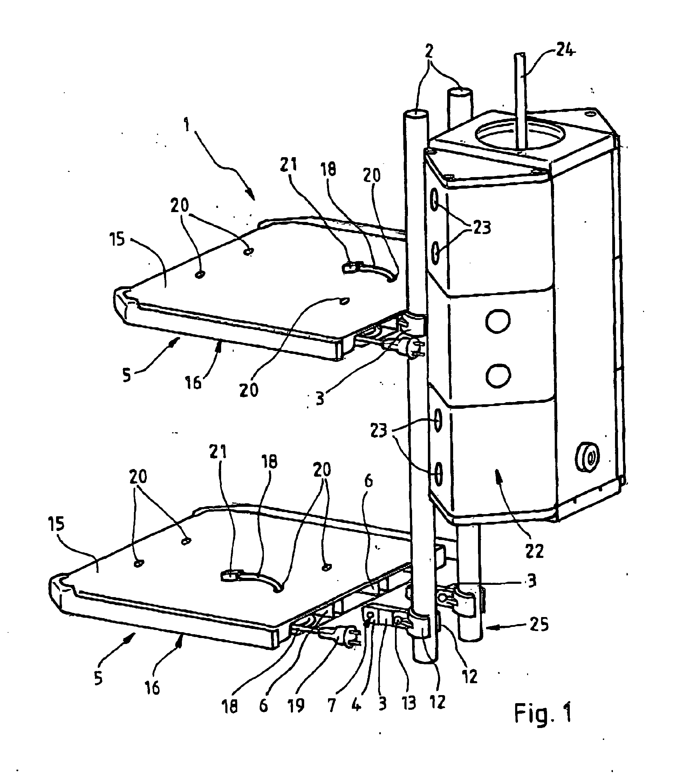

- Figur 1: in einer perspektivischen Ansicht eine Konsole mit zwei vertikalen Tragrohren und zwei Tabletts, in denen jeweils Stauräume zum Aufnehmen von Elektrokabeln vorgesehen sind, und die mittels jeweils zweier Steckverbindungen an den Tragrohren befestigt werden;

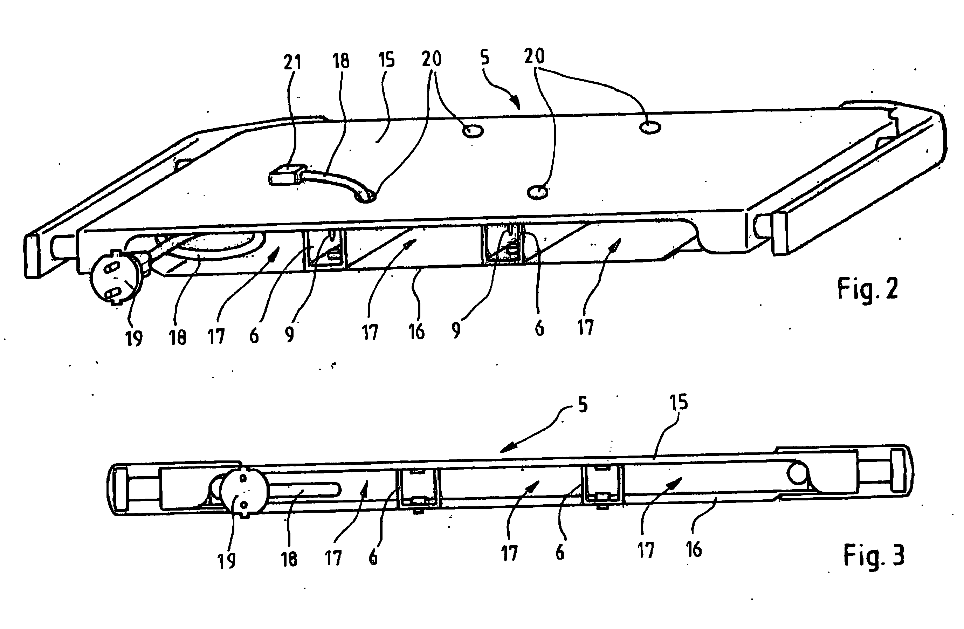

- Figur 2: in einer perspektivischen Ansicht ein Tablett der Figur 1;
- Figur 3: in einer Seitenansicht das Tablett der Figur 2;

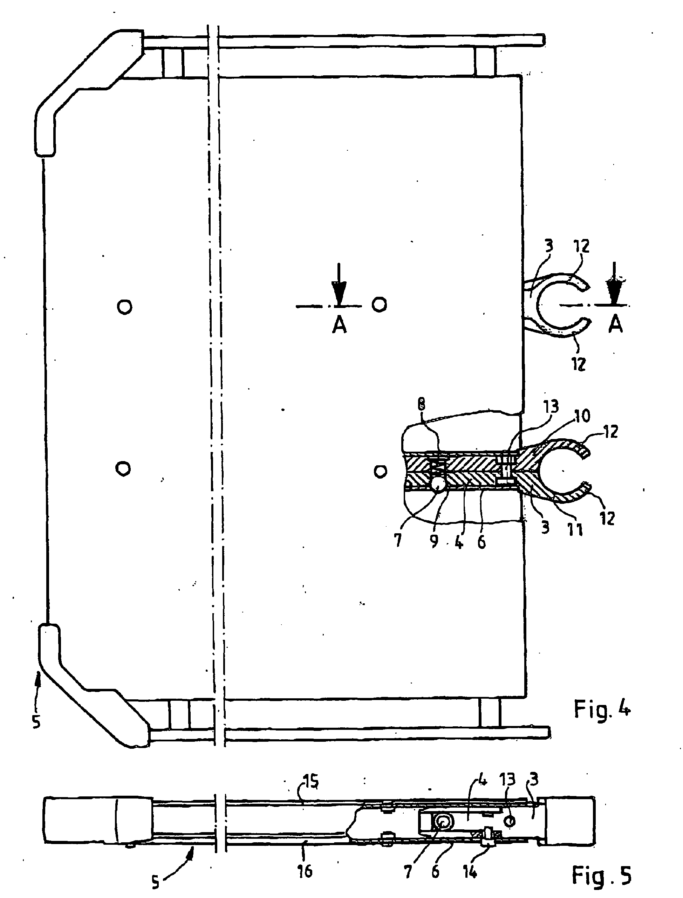

- Figur 4: in einer Ansicht von oben mit teilweisem Schnitt das Tablett der Figur 2, jedoch ohne Elektrokabel und mit eingesteckten Steckverbindungen, sowie
- Figur 5: in einer um 90 Grad zur Ansicht der Figur 3 versetzten Seitenansicht mit teilweisem Schnitt das Tablett der Figur 4.

Bei einer Konsole 1 für den Klinikbereich, mit zwei vertikalen Tragrohren 2, und mit vier Montageklauen 3, sind die Montageklauen 3 derart an den Tragrohren 2 befestigt, dass jeweils zwei Montageklauen 3 nebeneinander an den Tragrohren 2 vorgesehen sind. An den Montageklauen 3 ist jeweils eine Steckverbindung 4 ausgebildet. An jedem von zwei Tabletts sind jeweils zwei als Rechteckrohr ausgeführten Aufnahmen 6 zum jeweiligen Aufnehmen einer Steckverbindung 4 vorgesehen, um derart das Tablett auf die beiden Montageklauen 3 stecken zu können.

Die Steckverbindung 4 weist eine Kugel 7 auf, die in der Steckverbindung 4 gehalten wird, die mittels einer Feder 8 gegenüber der Steckverbindung 4 vorgespannt ist. Die Kugel 7 ist zum Einrasten in eine Aussparung 9 der Aufnahme 6 vorgesehen.

Die Montageklaue 3 besteht aus zwei Klauenteilen 10, 11, die jeweils ein Greifelement 12 aufweisen. Die Klauenteile 10, 11 sind mittels eines Verbindungselementes 13, welches als Schraube ausgeführt ist, miteinander verbunden. Ein Sicherungselement 14, welches eine Schraube ist, ist zum Sichern der Steckverbindung 4 in der Aufnahme 6 vorgesehen.

Jedes Tablett 5 weist eine obere Wandung 15 und eine untere Wandung 16 auf. Es sind jeweils zwei Aufnahmen 6 und drei Stauräume 17 zwischen den beiden Wandungen 15, 16 eines Tabletts vorgesehen. Die Stauräume 17 sind zur Aufnahme von Elektrokabeln 18 mitsamt deren Netzstecker 19 vorgesehen Dabei wird der Netzstecker 19 in den Stauraum 17 eingeklemmt. Der Wandungsabstand ist entsprechend bemessen. In der oberen Wandung 15, angrenzend an den linken und den mittleren Stauraum 17 sind insgesamt vier Aussparungen 20 zum, jeweiligen Durchführen eines später mit einer Anschlussbuchse 21 zu versehenen Elektrokabels 18 vorgesehen. Die Anschlussbuchse 21 dient zum Anschließen eines medizinischen Gerätes. An der Konsole 1 ist ein Korpus 22 vorgesehen, der eine Mehrzahl an Steckdosen 23 aufweist, Die Steckdosen 23 werden über eine elektrische Versorgungsleitung 24 mit elektrischen Strom versorgt.
- 1: Konsole
- 2: Tragrohr
- 3: Montageklaue
- 4: Steckverbindung
- 5: Tablett
- 6: Aufnahme
- 7: Kugel
- 8: Feder
- 9: Aussparung
- 10, 11: Klauenteil
- 12: Greifelement
- 13: Verbindungselement
- 14: Sicherungselement
- 15, 16: Wandung
- 17: Stauraum
- 18: Elektrokabel
- 19: Netzstecker
- 20: Aussparung
- 21: Anschlussbuchse
- 22: Korpus
- 23: Steckdose
- 24: Versorgungsleitung
- 25: Montageeinrichtung

## Patentansprüche

1. Konsole (1), insbesondere für den Klinikbereich, mit zwei vertikalen Tragrohren (2), und mit mindestens zwei Montageklauen (3), wobei die Montageklauen (3) derart an den Tragrohren (2) befestigt sind, dass zwei Montageklauen (3) nebeneinander an den Tragrohren (2) vorgesehen sind, **dadurch gekennzeichnet, dass** an den Montageklauen (3) jeweils eine Steckverbindung (4) ausgebildet ist, sowie mit mindestens einem Tablett (5), welches zwei Aufnahmen (6) zum jeweiligen Aufnehmen einer Steckverbindung (4) aufweist, um derart ein Tablett (5) auf die beiden Montageklauen (3) stecken zu können.

2. Konsole nach Anspruch 1, **dadurch gekennzeichnet, dass** als Aufnahme (6) ein Rechteckrohr vorgesehen ist.

3. Konsole nach Anspruch 1 oder Anspruch 2, **dadurch gekennzeichnet, dass** die Steckverbindung (4) eine Kugel (7) aufweist, die in der Steckverbindung (4) gehalten wird, die mittels einer Feder (8) gegenüber der Steckverbindung (4) vorgespannt ist, und die zum Einrasten in eine Aussparung (9) der Aufnahme (6) vorgesehen ist.

4. Konsole nach Anspruch 1, Anspruch 2 oder Anspruch 3, **dadurch gekennzeichnet, dass** die Montageklaue (3) aus zwei Klauenteilen (10, 11) besteht, die jeweils ein Greifelement (12) aufweisen, und dass die Klauenteile (10, 11) mittels eines Verbindungselementes (13), vorzugsweise einer Schraube, mitteinander verbunden sind.

5. Konsole nach mindestens einem der Ansprüch 1 bis 4, **dadurch gekennzeichnet, dass** ein Sicherungselement (14), vorzugsweise eine Schraube, zum Sichern der Steckverbindung (4) in der Aufnahme (6) vorgesehen ist.

6. Konsole mit mindestens einem der Ansprüche 1 bis 5, **dadurch gekennzeichnet, dass** das Tablett (5) eine obere Wandung (15) und eine untere Wandung (16) aufweist, und dass die Aufnahmen (6) zwischen den beiden Wandungen (15, 16) vorgesehen sind.

7. Konsole nach Anspruch 6, **dadurch gekennzeichnet, dass** zwischen den Wandungen (15, 16) mindestens ein Stauraum (17) vorgesehen ist.

8. Konsole nach Anspruch 7, **dadurch gekennzeichnet, dass** der Stauraum (17) zur Aufnahme eines Elektrokabels (18), vorzugsweise mitsamt eines Netzsteckers (19) vorgesehen ist.

9. Konsole nach Anspruch 8, **dadurch gekennzeichnet, dass** in der oberen Wandung (15), angrenzend an einen Stauraum (17), mindestens eine Aussparung (20) zum Durchführen eines mit einer Anschlussbuchse (21) versehenen Elektrokabels (18) vorgesehen ist.

10. Konsole nach Anspruch 9, **dadurch gekennzeichnet, dass** an der Konsole (1) ein Korpus (22) vorgesehen ist, der eine Mehrzahl an Steckdosen (23) aufweist, die über eine elektrische Versorgungsleitung (24) mit elektrischem Strom versorgt werden.

## Claims

1. Console (1) in particular for the hospital sector, comprising two vertical support tubes (2), with at least two assembly claws (3), in which the assembly claws (3) are attached to the support tubes (2) in such a way, that two assembly claws (3) are provided next to each other on the support tubes (2), wherein each of the assembly claws (3) has a plug connection (4), as well as at least one tray (5) which has two receptacles (6) for the respective accommodation of the plug connections (4) to allow the insertion of a tray (5) onto both of the assembly claws (3).

2. Console according to claim 1, wherein a rectangular tube is provided as receptacle (6).

3. Console according to claim 1 or claim 2, wherein the plug connection (4) has a ball (7), which is held in the plug connection (4), which is pre-loaded against the plug connection using a spring (8) and which is provided for latching into the recess of the receptacle (6).

4. Console according to claim 1, claim 2, or claim 3, wherein the assembly claw (3) consists of two claw parts (10, 11), each of which has a gripping element (12) and wherein the claw parts (10, 11) are connected to each other using a connection element (13), preferably a screw.

5. Console according to claim 1 to 4, wherein a retaining element (14) preferably a screw, is provided to secure the plug connection (4) in the receptacle (6).

6. Console according at least to one of the claims 1 to 5, wherein the tray (5) has an upper wall (15) and a lower wall (16), and wherein the receptacles (6) are provided between the two walls (15, 16).

7. Console according to claim 6, wherein at least one storage space (17) is provided between the walls (15, 16).

8. Console according to claim 7, wherein the storage space (17) is provided to accommodate an electric cable (18) preferably along with a power plug (19).

9. Console according to claim 8, wherein in the upper wall (15), bordering a storage space (17), at least one opening (20) is provided for routing an electric cable (18), which has a connector socket (21).

10. Console according to claim 9, wherein a body (22) is provided on the console, and the body has a multitude of power outlets (23) which are supplied with electric current via an electric supply line (24).

## Revendications

1. Console (1) en particulier pour le secteur clinique, comprenant deux tubes porteurs verticaux (2) et au moins deux pinces de montage (3), lesdites pinces de montage (3) étant fixées sur les tubes porteurs (2) de telle façon que deux pinces de montage (3) sont prévues l'une à côté de l'autre sur les tubes porteurs (2),
**caractérisée en ce qu'**un dispositif de liaison enfichage (4) est réalisé respectivement sur les pinces de montage (3), et avec au moins une tablette (5) qui comprend deux récepteurs (6) pour recevoir respectivement un dispositif de liaison à enfichage (4) afin de pouvoir enficher ainsi une tablette (5) sur les deux pinces de montage (3).

2. Console selon la revendication 1, **caractérisée en ce qu'**il est prévu un tube rectangulaire à titre de récepteur (6).

3. Console selon la revendication 1 ou 2, **caractérisée en ce que** le dispositif de liaison à enfichage (4) comprend une bille (7), qui est maintenue dans le dispositif de liaison à enfichage, qui est précontrainte au moyen d'un ressort (8) par rapport au dispositif de liaison enfichage (4), et qui est prévue pour s'enclencher dans une échancrure (9) du récepteur (6).

4. Console selon la revendication 1, 2 ou 3, **caractérisée en ce que** la pince de montage (3) est constituée de deux parties de pinces (10, 11) qui comprennent chacune un élément préhenseur (12), et **en ce que** les parties de pinces (10, 11) sont reliées l'une à l'autre au moyen d'un élément de liaison (13), de préférence d'une vis.

5. Console selon l'une au moins des revendications 1 à 4, **caractérisée en ce qu'**un élément de sécurité (14), de préférence une vis, est prévu pour bloquer la liaison à enfichage (6) dans le récepteur (6).

6. Console selon l'une au moins des revendications 1 à 5, **caractérisée en ce que** la tablette (5) comporte une paroi supérieure (15) et une paroi inférieure (16), et **en ce que** les récepteurs (6) sont prévus entre les deux parois (15, 16).

7. Console selon la revendication 6, **caractérisée en ce qu'**au moins un espace de rangement (17) est prévu entre les parois (15, 16).

8. Console selon la revendication 7, **caractérisée en ce que** l'espace de rangement (17) est prévu pour recevoir un câble électrique (18), de préférence conjointement avec une prise au secteur (19).

9. Console selon la revendication 8, **caractérisée en ce qu'**au moins une échancrure (20) est prévue dans la paroi supérieure (15) adjacente à un espace de rangement (17), pour la traversée d'un câble électrique (18) doté d'une douille de connexion (21).

10. Console selon la revendication 9, **caractérisé en ce que** sur la console (1) il est prévu un corps (22) qui comprend une pluralité de prises à enfichage (23), lesquelles sont alimentées en courant électrique via une ligne d'alimentation électrique (24).
